# EUROPÄISCHE PATENTSCHRIFT

(11) **EP 3 473 290 B1**
(45) Veröffentlichungstag und Bekanntmachung des Hinweises auf die Patenterteilung: **10.06.2020**
(21) Anmeldenummer: 18198083.0
(22) Anmeldetag: 02.10.2018
(51) Int. Cl.: A61M 16/08, F16L 5/10

(54) **MEDIZINGERÄT MIT ZUMINDEST EINER ABDICHTUNGSVORRICHTUNG**
MEDICAL DEVICE WITH AT LEAST ONE SEALING DEVICE
APPAREIL MÉDICAL DOTÉ D'AU MOINS UN DISPOSITIF D'ÉTANCHÉITÉ

(30) Priorität: 17.10.2017 DE 102017009674
(43) Veröffentlichungstag der Anmeldung: 24.04.2019
(73) Patentinhaber: Drägerwerk AG & Co. KGaA, 23558 Lübeck (DE)
(72) Erfinder: Elfring, Robert, 34131 Kassel (DE); Jäke, Christian, 20535 Hamburg (DE); Martin, Thomas, 23558 Lübeck (DE)

(56) Entgegenhaltungen:
- DE-A1- 10 355 213
- DE-A1-102010 048 518

## Beschreibung

Die Erfindung betrifft ein Medizingerät mit einer Anschlussstelle zwischen einem Kopplungselement und einem an das Kopplungselement angeschlossenen Leitungselement sowie einer Vorrichtung zum Abdichten der Verbindungsstelle (Abdichtungsvorrichtung). Bei dem Kopplungselement handelt es sich zum Beispiel um ein als Gasentnahmestelle fungierendes Kopplungselement.

Gasbereiche sind - unter anderem in der Medizintechnik - entsprechend geltender Normen räumlich von Elektronik- oder Elektrikbereichen zu trennen. Dies gilt speziell für medizinische Versorgungseinheiten. Eine solche Vorschrift basiert auf dem Umstand, dass zum Beispiel eine erhöhte Sauerstoffkonzentration in Verbindung mit elektrischen Potenzialen zu einem Brand oder einer Explosion führen können.

Zur Trennung eines Gasbereichs von Elektronik- oder Elektrikbereichen, also Bereichen mit elektrischen und/oder elektronischen Schaltungen oder Verbrauchern, wird aktuell entweder eine horizontale Bereichsabschottung oder eine vertikale Bereichsabschottung verwendet. Bei einer horizontalen Bereichsabschottung macht man sich zunutze, dass austretendes Gas, zum Beispiel Sauerstoff (O₂), schwerer als die Umgebungsluft ist und in einem abgeschotteten Bereich unter Gravitationseinfluss nach unten sinkt, wo zum Beispiel über eine Ventilationsöffnung am Boden des Geräts eine Vermischung des Gases mit der Umgebungsluft erfolgt. Als Gasbereich fungiert folglich ein durch ein horizontales Schottblech von einem Elektronik- oder Elektrikbereich getrennter Bereich, der räumlich unterhalb des Elektronik- oder Elektrikbereichs angeordnet ist. Bei einer vertikalen Bereichsabschottung trennt ein vertikales Schottblech den Gasbereich vom Elektronik- oder Elektrikbereich, wobei auch hier austretendes Gas, das schwerer als die Umgebungsluft ist, in dem abgeschotteten Bereich unter Gravitationseinfluss nach unten sinkt.

Aktuell ist eine solche Trennung durch Ventilationsöffnungen, Schottbleche oder komplett getrennte Kanäle realisiert. Die ISO11197, der partikuläre Sicherheitsstandard für medizinische Versorgungseinheiten, schreibt in der aktuell verifizierten Version (Stand 2004) eine Trennung/Schottung sowie eine Ventilation innerhalb der Versorgungseinheit vor, um einer Sauerstoffanreicherung entgegenzuwirken. Die ISO11197, Stand 2004 erlaubt eine nahezu freie Positionierung von Gasentnahmestellen zur Entnahme brennbarer Gase, insbesondere O₂ und N₂O. Allerdings schreibt die ISO11197, Stand 2016 zusätzlich zu den Anforderungen gemäß dem Stand aus 2004 eine definierte Leckage von 1 I O₂ pro Minute für mindestens 10 min Einströmzeit vor. Hierdurch wird die freie Positionierung von Gasentnahmestellen zur Entnahme brennbarer Gase stark eingeschränkt, denn durch die Definition der Leckagegröße ist die Positionierung einer Gasentnahmestelle zum Beispiel über einer Steckdose nicht mehr möglich. Das große O₂-Volumen sorgt für einen ansteigenden O₂-Wert über einen kritischen Wert von zum Beispiel 25 Vol.-%. Zulässige Positionierungen sind dann nur noch eingeschränkt möglich. Zum Beispiel durch vertikale Bereichsabschottungen und vertikale Positionierung (Gas neben Elektro) oder horizontale Bereichsabschottung und horizontale Positionierung (Elektro über Gas).

DE 10 2010 048 518 A1 zeigt eine Vorrichtung, um mittels eines Rohrleitungssystems 17 Druckgase in einer medizinischen Behandlungseinrichtung zu verteilen. Ein metallisches Rahmengehäuse 12 wird von einer Brandschutzschalung 1 aufgenommen. Die Brandschutzschalung 1 umfasst seitliche Rahmenplatten 2, 3, 4, 5, eine Rückenplatte 6 und eine Deckplatte 7, welche zusammen einen Hohlraum 8 umschließen. Elektroleitungen 11 sind durch Durchbrüche 10 in der unteren Rahmenplatte 4 hindurch geführt. Rohrleitungen 17 sind durch Durchbrüche 16 in der oberen Rahmenplatte 5 hindurch geführt. Der Hohlraum 8 ist mit feuerfesten Materialien ausgefüllt, welche sich an die Außenwanderungen der Rohrleitungen anlegen, sodass eine Gasdichtigkeit zum Innenraum des metallischen Rahmengehäuses hergestellt werden kann.

In DE 103 55 213 A1 wird beschrieben, wie eine Rohrleitung 1 durch den Mantel 2 einer Coldbox hindurch geführt wird. Ein Balg mit zwei Balgenden 4 und 5 sowie ein schlauchförmiges Mittelteil 6 ist aus einem Aramid-Gewebe gefertigt, umgibt die Rohrleitung 1 und ist über einen Stutzen 3 an dem Mantel 2 geschoben. Eine Pufferschicht 9 zwischen dem Balgende fünf unter Rohrleitung 1 bildet Hohlräume aus, welche das Ara Mietgewebe des Balkens thermisch von der Rohrleitung 1 isoliert und die unterschiedlich starke thermische Ausdehnung des Aramid-Gewebes und der Rohrleitung 1 kompensiert. Jeweils eine Zwischenlage 8 aus Glasgewebe ist zwischen dem Stutzen 3 und dem Balgende 4 sowie zwischen dem Balgende 4 und einer Schelle 7 um den Stutzen 3 gelegt.

Eine Aufgabe der vorliegenden Erfindung besteht darin, eine Möglichkeit anzugeben, mittels derer Brand- und/oder Explosionsgefahren aufgrund von ausströmenden Gas sicher vermeidbar sind, insbesondere darin, eine Möglichkeit anzugeben, bei Vermeidung von Brand- und/oder Explosionsgefahren Gasentnahmestellen im Wesentlichen frei positionieren zu können.

Die Neuerung betrifft ein Medizingerät, insbesondere ein als medizinische Versorgungseinheit fungierendes Medizingerät, mit zumindest einem Kopplungselement und einem an das Kopplungselement angeschlossenen Leitungselement, wobei zumindest ein Teil des Kopplungselements sowie zumindest ein Abschnitt des Leitungselements mittels einer Abdichtungsvorrichtung der hier und im Folgenden beschriebenen Art eingeschlossen sind.

Erfindungsgemäß wird diese Aufgabe mittels dieses Medizingeräts umfassend eine gasdichte Abdichtungsvorrichtung mit den Merkmalen des Anspruchs 1 gelöst. Dazu ist bei einer solchen Abdichtungsvorrichtung vorgesehen, dass diese einerseits zumindest einen Teil eines Kopplungselements oder einen Teil einer Wandung mit dem Kopplungselement sowie andererseits zumindest einen Abschnitt eines an das Kopplungselement angeschlossenen Leitungselements einschließt, nämlich gasdicht einschließt. Für diesen gasdichten Einschluss schließt die Abdichtungsvorrichtung an einer ersten Abdichtstelle gasdicht allseitig umlaufend an das Leitungselement an und an einer zweiten Abdichtstelle gasdicht allseitig umlaufend an das Kopplungselement oder eine Wandung, in oder an der das Kopplungselement angebracht ist, an. Zwischen der ersten Abdichtstelle und der zweiten Abdichtstelle schließt die Abdichtungsvorrichtung ein durch die Geometrie der Abdichtungsvorrichtung bestimmtes Volumen gasdicht ein.

Bei dem Kopplungselement handelt es sich um eine Vorrichtung zur Weiterleitung eines ankommenden Gasstroms in ein an das Kopplungselement anschließbares Leitungselement. Als Kopplungselement fungiert zum Beispiel eine Rohrverbindung in Form einer Schlauchanschlusstülle, wobei das Leitungselement an einen zum Aufstecken eines Endes des Leitungselements bestimmten Abschnitt angeschlossen wird. Das Leitungselement ist zur Weiterleitung des Gasstroms bestimmt und eingerichtet und bei dem Leitungselement handelt es sich zum Beispiel um einen Schlauch oder dergleichen.

Der Vorteil der Erfindung besteht darin, dass die Abdichtungsvorrichtung mit ihrem Volumen zwischen der ersten und der zweiten Abdichtstelle eventuell an der Verbindungsstelle zwischen dem Leitungselement und dem Kupplungselement ausströmendes Gas auffängt und gasdicht einschließt. Eine Brand- oder Explosionsgefahr aufgrund von ausströmendem Gas ist damit sicher vermieden, denn ausströmendes Gas kann nicht in den Bereich elektrischer oder elektronischer Schaltungen oder Verbraucher gelangen. Aufgrund der erreichten Vermeidung der Brand-und Explosionsgefahr kann ein als Gasentnahmestelle fungierendes Kopplungselement im Grunde beliebig positioniert werden, also zum Beispiel auch in räumlicher Nähe zu elektrischen oder elektronischen Schaltungen oder Verbrauchern. Dies erleichtert die Konstruktion von Geräten, zum Beispiel Medizingeräten, insbesondere Medizingeräten in Form von medizinischen Versorgungseinheiten, welche zumindest eine Gasentnahmestelle umfassen, und erlaubt zum Beispiel eine kompaktere Bauform, weil kein besonderer Abstand zwischen einer Gasentnahmestelle und elektrischen oder elektronischen Schaltungen oder Verbrauchern eingehalten werden muss.

Vorteilhafte Ausgestaltungen der Erfindung sind Gegenstand der Unteransprüche. Dabei verwendete Rückbeziehungen weisen auf die weitere Ausbildung des Gegenstandes des Hauptanspruches durch die Merkmale des jeweiligen Unteranspruches hin und sind nicht als ein Verzicht auf die Erzielung eines selbständigen, gegenständlichen Schutzes für die Merkmalskombinationen der rückbezogenen Unteransprüche zu verstehen. Des Weiteren ist im Hinblick auf eine Auslegung der Ansprüche sowie der Beschreibung bei einer näheren Konkretisierung eines Merkmals in einem nachgeordneten Anspruch davon auszugehen, dass eine derartige Beschränkung in den jeweils vorangehenden Ansprüchen sowie einer allgemeineren Ausführungsform des gegenständlichen Einschraubverbinders nicht vorhanden ist. Jede Bezugnahme in der Beschreibung auf Aspekte nachgeordneter Ansprüche ist demnach auch ohne speziellen Hinweis ausdrücklich als Beschreibung optionaler Merkmale zu lesen.

Bei einer Ausführungsform der Abdichtungsvorrichtung ist diese einstückig aus einem randseitig als Dichtung fungierenden Material, zum Beispiel einem Elastomer, hergestellt. Die resultierende Flexibilität der Abdichtungsvorrichtung erleichtert deren Handhabung und Anbringung. Die Einstückigkeit vermeidet die Verwendung einer ansonsten notwendigen Dichtung und erleichtert damit ebenfalls die Handhabung und Anbringung.

Bei einer weiteren Ausführungsform der Abdichtungsvorrichtung weist diese einen Entlüftungsanschluss zwischen der ersten Abdichtstelle und der zweiten Abdichtstelle auf. Über den Entlüftungsanschluss ist ein Druckausgleich zwischen dem Innern der Abdichtungsvorrichtung und der Umgebung sowie eine Fortleitung von mittels der Abdichtungsvorrichtung aufgefangenem Gas möglich. An den Entlüftungsanschluss kann ein Leitungselement in Form eines Schlauchs oder dergleichen angeschlossen sein, um bei einer Fortleitung von aufgefangenem Gas dieses an einem Ort abzulassen, an dem aufgrund des Gases keine Brand- oder Explosionsgefahr besteht.

Bei einer besonderen Ausführungsform der Abdichtungsvorrichtung ist diese geometrisch und konstruktiv in einer Art und Weise gestaltet, die zu einer kompakten, nur ein geringes Volumen beanspruchenden Bauform führt. Dazu ist vorgesehen, dass die Abdichtungsvorrichtung außer dem Kopplungselement oder dem Teil des Kopplungselements sowie dem Teil des Leitungselements keine weiteren Komponenten einschließt. Dann können die Wandlungen der Abdichtungsvorrichtung eng an die Außenoberflächen des Kopplungselements und des Leitungselements anschließen. Eine solche kompakte Bauform führt dazu, dass eine Gasentnahmestelle mit einer umgebenden Abdichtungsvorrichtung nur unwesentlich mehr Bauraum beansprucht als eine Gasentnahmestelle ohne eine solche Abdichtungsvorrichtung. Dies erleichtert die Konstruktion von Geräten, insbesondere Medizingeräten, mit zumindest einer mittels einer Abdichtungsvorrichtung der hier und im Folgenden beschriebenen Art eingehausten Gasentnahmestelle.

Nachfolgend wird ein Ausführungsbeispiel der Erfindung anhand der Zeichnung näher erläutert. Einander entsprechende Gegenstände oder Elemente sind in allen Figuren mit den gleichen Bezugszeichen versehen.

Das Ausführungsbeispiel ist nicht als Einschränkung der Erfindung zu verstehen. Vielmehr sind im Rahmen der vorliegenden Offenbarung Abänderungen und Modifikationen möglich, insbesondere solche Varianten und Kombinationen, die zum Beispiel durch Kombination oder Abwandlung von einzelnen in Verbindung mit den im allgemeinen oder speziellen Beschreibungsteil beschriebenen sowie in den Ansprüchen und/oder der Zeichnung enthaltenen Merkmalen für den Fachmann im Hinblick auf die Lösung der Aufgabe entnehmbar sind und durch kombinierbare Merkmale zu einem neuen Verfahren oder Gegenstand führen.

Es zeigen:
- Figur 1: eine ein Kopplungselement sowie einen Schlauchabschnitt umschließende und gasdicht einschließende Abdichtungsvorrichtung,
- Figur 2: die Abdichtungsvorrichtung gemäß Figur 1 mit einem an die Abdichtungsvorrichtung angeschlossenen ableitenden Leitungselement,
- Figur 3: eine Abdichtungsvorrichtung wie in Figur 2, wobei die Abdichtungsvorrichtung an das Kopplungselement anschließt, und
- Figur 4: eine Abdichtungsvorrichtung, die eine Mehrzahl von an ein Kopplungselement angeschlossenen Schlauchabschnitten und das Kopplungselement umschließt und gasdicht einschließt.

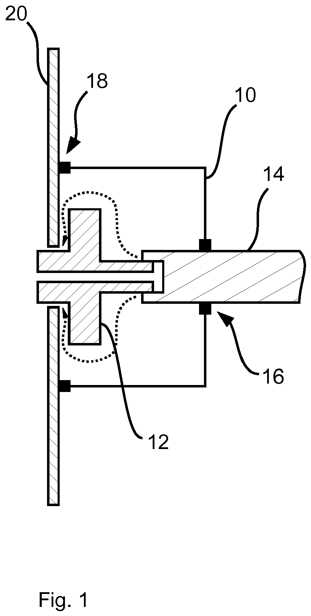

Die Darstellung in Figur 1 zeigt in Form einer schematisch vereinfachten Schnittdarstellung eine Ausführungsform einer gegenständlichen Abdichtungsvorrichtung 10. Diese umschließt gasdicht ein zum Beispiel als Entnahmestelle (Gasentnahmestelle) für brennbares Gas fungierendes Kopplungselement 12, insbesondere ein Kopplungselement 12 in Form von oder nach Art einer Schlauchanschlusstülle, sowie einen Abschnitt eines an das Kopplungselement 12 angeschlossenen Leitungselements 14, also zum Beispiel einen Abschnitt eines Schlauchs. Dafür schließt die Abdichtungsvorrichtung 10 an einer ersten Abdichtstelle 16 gasdicht allseitig umlaufend an das Leitungselement 14 an und umfasst dafür zum Beispiel eine Dichtung, welche sich in grundsätzlich an sich bekannter Art und Weise im Bereich der ersten Abdichtstelle 16 an die Außenoberfläche des Leitungselements 14 anlegt und somit für einen gasdichten Abschluss sorgt. Ebenso schließt die Abdichtungsvorrichtung 10 an einer zweiten Abdichtstelle 18 gasdicht allseitig umlaufend entweder an das Kopplungselement 12 oder - wie hier gezeigt - an eine Wandung 20, zum Beispiel eine Gehäusewand, eine Gebäudewand oder dergleichen, nämlich eine Wandung 20, in oder an der das Kopplungselement 12 angebracht ist, an. Auch hier umfasst die Abdichtungsvorrichtung 10 für den gasdichten Anschluss zum Beispiel eine Dichtung, welche sich in grundsätzlich an sich bekannter Art und Weise im Bereich der zweiten Abdichtstelle 18 an die Oberfläche der Wandung 20 anlegt. Anstelle einer Dichtung im Bereich der ersten und/oder zweiten Abdichtstelle 16, 18 kann auch ein gasdichter Anschluss der Abdichtungsvorrichtung 10 an das Leitungselement 14 bzw. das Kopplungselement 12 oder die Wandung 20 mittels einer Klebverbindung erfolgen. Eine solche Klebverbindung hat den Vorteil, dass diese unmittelbar auch dafür wirksam ist, die Abdichtungsvorrichtung 10 insgesamt ortsfest zu fixieren. Bei einer Dichtung an der ersten und/oder zweiten Absichtsstelle 16,18 ist gegebenenfalls noch eine zusätzliche mechanische Fixierung der Abdichtungsvorrichtung 10 erforderlich, welche diese in Position, also in gasdichtem Kontakt zum Beispiel mit der Wandung 20 hält.

Die Abdichtungsvorrichtung 10 bewirkt einen gasdichten Einschluss der Verbindungsstelle zwischen dem Leitungselement 14 und dem Kopplungselement 12. Bei einer angebrachten Abdichtungsvorrichtung 10 gelangt das im Falle eines undichten Anschlusses des Leitungselements 14 an das Kopplungselement 12 ausströmende Gas nur in das Innere der Abdichtungsvorrichtung 10. Von dort kann das Gas gegebenenfalls über verbleibende Spalte zwischen dem Kopplungselement 12 und der Wandung 20 abfließen, wie dies in der Darstellung in Figur 1 durch die von dem an das Kopplungselement 12 angeschlossenen Ende des Leitungselements 14 ausgehenden Pfeile gezeigt ist.

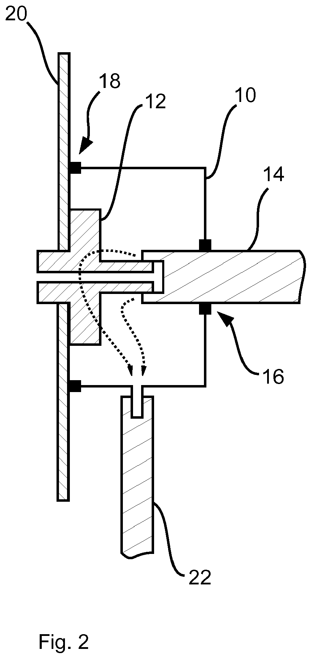

Die Darstellung in Figur 2 zeigt eine Ausführungsform der Abdichtungsvorrichtung 10 gemäß Figur 1, bei der diese einen Entlüftungsanschluss für ein im Folgenden zur Unterscheidung von dem Leitungselement 14 - aber ohne Verzicht auf eine weitergehende Allgemeingültigkeit - als Schlauch 22 bezeichnetes ableitendes Leitungselement aufweist. Gas, das im Falle eines undichten Anschlusses des Leitungselements 14 an das Kopplungselement 12 ausströmt und zunächst in das Innere der Abdichtungsvorrichtung 10 gelangt, kann über den Schlauch 22 abfließen. Allgemein fungieren der Entlüftungsanschluss und ein ggf. angeschlossener Schlauch 22 als Entlüftung der Abdichtungsvorrichtung 10.

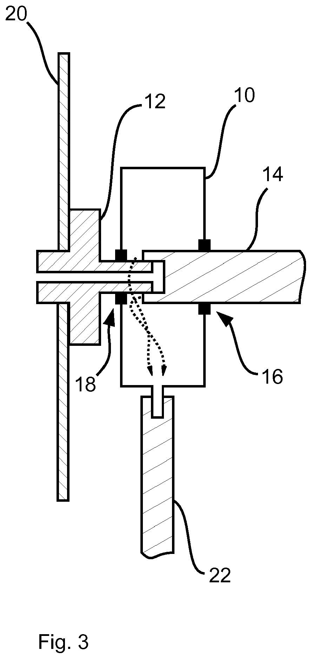

Die Darstellung in Figur 3 zeigt eine Ausführungsform, bei der die Abdichtungsvorrichtung 10 einerseits an das Leitungselement 14 und andererseits an das Kopplungselement 12 anschließt und so für einen gasdichten Einschluss der Verbindungsstelle zwischen dem Leitungselement 14 und dem Kopplungselement 12 sorgt. Die Erläuterungen im Zusammenhang mit Figur 1 und Figur 2 gelten für die in Figur 3 gezeigte Ausführungsform der Abdichtungsvorrichtung 10 entsprechend, sodass hier auf unnötige Wiederholungen verzichtet werden kann. Im Unterschied zu der Ausführungsform in Figur 1 und Figur 2 schließt die Abdichtungsvorrichtung 10 an einer zweiten Abdichtstelle 18 gasdicht allseitig umlaufend an das Kopplungselement 12 an.

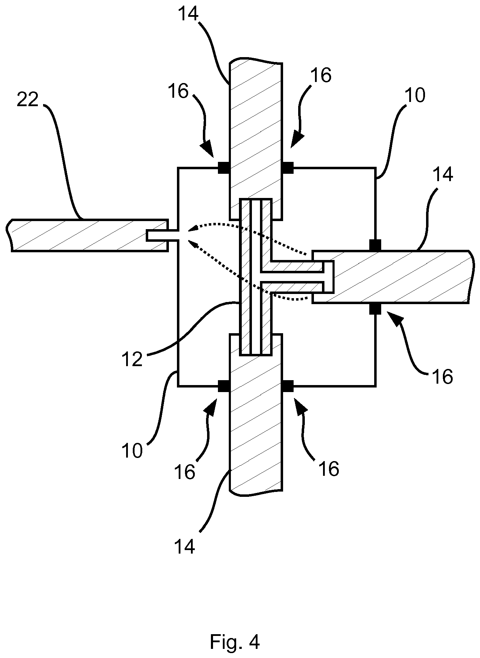

Die Darstellung in Figur 4 zeigt eine Ausführungsform, bei der die Abdichtungsvorrichtung 10 mehrere Verbindungsstellen zwischen einem Kopplungselement 12 und jeweils einem Leitungselement 14 gasdicht einschließt. Die Abdichtungsvorrichtung 10 weist eine der Anzahl der Leitungselemente 14 entsprechende Anzahl von ersten Abdichtstellen 16 auf, an denen die Abdichtungsvorrichtung 10 gasdicht allseitig umlaufend an das jeweilige Leitungselement 14 anschließt. Bei der gezeigten Ausführungsform weist diese einen grundsätzlich optionalen Entlüftungsanschluss auf und über diesen Entlüftungsanschluss ist in ebenfalls grundsätzlich optionaler Art und Weise an die Abdichtungsvorrichtung 10 ein Schlauch 22 zur Fortleitung von an einer Verbindungsstelle zwischen dem Kopplungselement 12 und einem Leitungselement 14 ausgetretenem und mittels der Abdichtungsvorrichtung 10 aufgefangenem Gas angeschlossen.

Für alle gezeigten Ausführungsformen gilt, dass in den jeweiligen Schnittdarstellungen nur ein Abschnitt der Wandung der Abdichtungsvorrichtung 10 sichtbar ist und dass demgemäß die Abdichtungsvorrichtung 10 gedanklich quer zur Blattebene in beiden Richtungen zu einem geschlossenen Gehäuse zu ergänzen ist. Bei der Abdichtungsvorrichtung 10 handelt es sich bevorzugt um ein einstückiges Kunststoffteil. Optional ist die Abdichtungsvorrichtung 10 elastisch und zum Beispiel einstückig aus einem randseitig als Dichtung fungierenden Material hergestellt, zum Beispiel einem Elastomer. Grundsätzlich kommt auch eine mehrteilige Abdichtungsvorrichtung 10 in Betracht. Dann weist die Abdichtungsvorrichtung 10 an den Verbindungsstellen zwischen den Einzelteilen eine Dichtung oder dergleichen für einen gasdichten Anschluss eines der Einzelteile an das andere Einzelteil auf.

Die Abdichtungsvorrichtung 10 gemäß Figur 1, Figur 2 und Figur 3 kann eine zylindrische Grundform haben, mit einer Hochachse parallel zu einem in der Abdichtungsvorrichtung 10 ganz oder teilweise eingeschlossenen Kopplungselement 12. Alternativ kann die Abdichtungsvorrichtung 10 auch eine polygonale Grundform haben, zum Beispiel eine würfelförmige oder quaderförmige Grundform. Grundsätzlich ist die äußere Form der Abdichtungsvorrichtung 10 beliebig und kann auch - zum Beispiel im Hinblick auf ein möglichst geringes Volumen und damit einen möglichst geringen Raumbedarf - beliebig komplex sein und zum Beispiel den Randlinien der jeweils eingehausten, im Innern der Abdichtungsvorrichtung 10 gekapselten Komponenten (Kopplungselement 12, Leitungselement 14) folgen.

Die Konfigurationen gemäß Figur 1 bis Figur 4 fungieren zum Beispiel als Gasentnahmestellen. Aufgrund des gasdichten Einschlusses der oder jeder grundsätzlich leckagegefährdeten Verbindungsstelle mittels der Abdichtungsvorrichtung 10 ist die Gasentnahmestelle insgesamt gasdicht eingeschlossen. Damit kann die Gasentnahmestelle grundsätzlich beliebig positioniert werden und sich zum Beispiel auch in der Nähe elektrischer oder elektronischer Schaltungen oder Verbraucher befinden.

Einzelne im Vordergrund stehende Aspekte der hier eingereichten Beschreibung lassen sich damit kurz wie folgt zusammenfassen: Angegeben wird eine gasdichte Abdichtungsvorrichtung 10, welche einerseits zumindest einen Teil eines Kopplungselements 12 oder einen Teil einer Wandung 20 mit dem Kopplungselement 12 sowie andererseits zumindest einen Abschnitt eines an das Kopplungselement 12 angeschlossenen Leitungselements 14 einschließt, wobei die Abdichtungsvorrichtung 10 an einer ersten Abdichtstelle 16 gasdicht allseitig umlaufend an das Leitungselement 14 anschließt, wobei die Abdichtungsvorrichtung 10 an einer zweiten Abdichtstelle 18 gasdicht allseitig umlaufend an das Kopplungselement 12 oder eine Wandung 20, in oder an der das Kopplungselement 12 angebracht ist, anschließt und wobei die Abdichtungsvorrichtung 10 zwischen der ersten Abdichtstelle 16 und der zweiten Abdichtstelle 18 ein durch die Geometrie der Abdichtungsvorrichtung 10 bestimmtes Volumen gasdicht einschließt. Die Abdichtungsvorrichtung 10 fungiert im Gegensatz zu der bisher im Stand der Technik vorgesehenen Bereichsabschottung als Einzelabschottung.

### BEZUGSZEICHENLISTE

- 10: Abdichtungsvorrichtung
- 12: Kopplungselement
- 14: Leitungselement
- 16: erste Abdichtstelle
- 18: zweite Abdichtstelle
- 20: Wandung
- 22: Schlauch (ableitendes Leitungselement)

## Patentansprüche

1. Medizingerät mit zumindest einem Kopplungselement (12) und einem an das Kopplungselement (12) angeschlossenen Leitungselement (14),
wobei zumindest ein Teil des Kopplungselements (12) sowie zumindest ein Abschnitt des Leitungselements (14) mittels einer gasdichten Abdichtungsvorrichtung (10) gasdicht eingeschlossen sind,
wobei die Abdichtungsvorrichtung (10) an einer ersten Abdichtstelle (16) gasdicht allseitig umlaufend an das Leitungselement (14) anschließt,
wobei die Abdichtungsvorrichtung (10) an einer zweiten Abdichtstelle (18) gasdicht allseitig umlaufend an das Kopplungselement (12) oder eine Wandung (20), in oder an der das Kopplungselement (12) angebracht ist, anschließt und
wobei die Abdichtungsvorrichtung (10) zwischen der ersten Abdichtstelle (16) und der zweiten Abdichtstelle (18) ein durch die Geometrie der Abdichtungsvorrichtung (10) bestimmtes Volumen gasdicht einschließt.

2. Medizingerät nach Anspruch 1, wobei die Abdichtungsvorrichtung (10) einstückig aus einem randseitig als Dichtung fungierenden Material hergestellt ist.

3. Medizingerät nach Anspruch 1 oder 2, wobei die Abdichtungsvorrichtung (10) einen Entlüftungsanschluss zwischen der ersten Abdichtstelle (16) und der zweiten Abdichtstelle (18) aufweist.

4. Medizingerät nach Anspruch 1, 2 oder 3, wobei die Abdichtungsvorrichtung (10) außer dem Kopplungselement (12) oder dem Teil des Kopplungselements (12) sowie dem Teil des Leitungselements (14) keine weiteren Komponenten einschließt.

## Claims

1. Medical appliance having at least one coupling element (12) and one line element (14) connected to the coupling element (12),
wherein at least one part of the coupling element (12) and at least one portion of the line element (14) are enclosed in a gas-tight manner by means of a gas-tight sealing device (10),
wherein the sealing device (10), at a first sealing location (16), connects circumferentially in a gas-tight manner, on all sides, to the line element (14),
wherein the sealing device (10), at a second sealing location (18), connects circumferentially in a gas-tight manner, on all sides, to the coupling element (12) or to a wall (20) in or at which the coupling element (12) is mounted, and
wherein the sealing device (10), between the first sealing location (16) and the second sealing location (18), encloses in a gas-tight manner a volume defined by the geometry of the sealing device (10).

2. Medical appliance according to Claim 1, wherein the sealing device (10) is produced in one piece from a material that functions as seal at the edge.

3. Medical appliance according to Claim 1 or 2, wherein the sealing device (10) has a ventilation port between the first sealing location (16) and the second sealing location (18).

4. Medical appliance according to Claim 1, 2 or 3, wherein the sealing device (10) encloses no further components than the coupling element (12) or the part of the coupling element (12) and the part of the line element (14) .

## Revendications

1. Appareil médical comprenant au moins un élément de couplage (12) et un élément de conduite (14) raccordé à l'élément de couplage (12), au moins une partie de l'élément de couplage (12) ainsi qu'au moins une portion de l'élément de conduite (14) étant fermées de manière étanche aux gaz au moyen d'un dispositif d'étanchéité étanche aux gaz (10), le dispositif d'étanchéité (10) se raccordant de manière étanche aux gaz à l'élément de conduite (14) de tous les côtés sur la périphérie au niveau d'une première zone d'étanchéité (16), le dispositif d'étanchéité (10) se raccordant, au niveau d'une deuxième zone d'étanchéité (18), de manière étanche aux gaz de tous les côtés sur la périphérie, à l'élément de couplage (12) ou à une paroi (20) dans laquelle ou au niveau de laquelle l'élément de couplage (12) est monté et le dispositif d'étanchéité (10), entre la première zone d'étanchéité (16) et la deuxième zone d'étanchéité (18), renfermant de manière étanche aux gaz un volume déterminé par la géométrie du dispositif d'étanchéité (10).

2. Appareil médical selon la revendication 1, dans lequel le dispositif d'étanchéité (10) est fabriqué d'une seule pièce à partir d'un matériau servant de joint d'étanchéité du côté du bord.

3. Appareil médical selon la revendication 1 ou 2, dans lequel le dispositif d'étanchéité (10) présente un raccord de désaérage entre la première zone d'étanchéité (16) et la deuxième zone d'étanchéité (18).

4. Appareil médical selon la revendication 1, 2 ou 3, dans lequel le dispositif d'étanchéité (10) ne renferme aucun composant supplémentaire à l'exception de l'élément d'accouplement (12) ou de la partie de l'élément d'accouplement (12) ainsi que de la partie de l'élément de conduite (14).
